# EUROPEAN PATENT SPECIFICATION

(11) **EP 4 103 939 B1**
(45) Date of publication and mention of the grant of the patent: **11.12.2024**
(21) Application number: 20842124.8
(22) Date of filing: 18.12.2020
(51) Int. Cl.: G01N 27/30, C25B 11/043

(54) **PH ELECTRODE WITH BORON DOPED DIAMOND REGION**
PH-ELEKTRODE MIT BORDOTIERTEM DIAMANTBEREICH
ÉLECTRODE DE PH AVEC RÉGION DE DIAMANT DOPÉE AU BORE

(30) Priority: 13.02.2020 US 202062976043 P
(43) Date of publication of application: 21.12.2022
(73) Proprietor: Hach Company, Loveland, CO 80538-8842 (US)
(72) Inventor: DUNCAN, Zoe, Daventry Northamptonshire NN114AJ (GB)
(74) Representative: Sandri, Sandro
(86) International application number: PCT/US2020/065970
(87) International publication number: WO 2021/162782

(56) References cited:
- ZO? J. AYRES ET AL: "Controlled sp 2 Functionalization of Boron Doped Diamond as a Route for the Fabrication of Robust and Nernstian pH Electrodes", ANALYTICAL CHEMISTRY, vol. 88, no. 1, 5 January 2016 (2016-01-05), pages 974 - 980, XP055246978, ISSN: 0003-2700, DOI: 10.1021/acs.analchem.5b03732
- TANIA L. READ ET AL: "In Situ Control of Local pH Using a Boron Doped Diamond Ring Disk Electrode: Optimizing Heavy Metal (Mercury) Detection", ANALYTICAL CHEMISTRY, vol. 86, no. 1, 7 January 2014 (2014-01-07), pages 367 - 371, XP055140575, ISSN: 0003-2700, DOI: 10.1021/ac403519p
- TANIA L. READ ET AL: "An sp 2 Patterned Boron Doped Diamond Electrode for the Simultaneous Detection of Dissolved Oxygen and pH", ACS SENSORS, vol. 4, no. 3, 22 February 2019 (2019-02-22), pages 756 - 763, XP055610398, ISSN: 2379-3694, DOI: 10.1021/acssensors.9b00137
- AYRES ZOË J ET AL: "Quinone electrochemistry for the comparative assessment of sp2surface content of boron doped diamond electrodes", ELECTROCHEMISTRY COMMUNICATIONS, ELSEVIER AMSTERDAM, NL, vol. 72, 31 August 2016 (2016-08-31), pages 59 - 63, XP029786081, ISSN: 1388-2481, DOI: 10.1016/J.ELECOM.2016.08.024
- ANTHONY J. LUCIO ET AL: "Investigation of sp 2 -Carbon Pattern Geometry in Boron-Doped Diamond Electrodes for the Electrochemical Quantification of Hypochlorite at High Concentrations", ACS SENSORS, vol. 5, no. 3, 11 February 2020 (2020-02-11), pages 789 - 797, XP055732646, ISSN: 2379-3694, DOI: 10.1021/acssensors.9b02444

## Description

### FIELD

This application relates generally to pH measurement of an aqueous sample, and, more particularly, to pH electrodes with a carbon region.

### BACKGROUND

The article "Controlled sp 2 Functionalization of Boron Doped Diamond as a Route for the Fabrication of Robust and Nernstian pH Electrodes", was published as doi:10.1021/acs.analchem.5b03732 on 06-12-2015, and relates to boron doped diamond (BDD) pH sensors. Ensuring water quality is critical to the health and well-being of humans, animals, and plants, which are reliant on water for survival. One parameter of water that may be measured is the pH. The measurement of pH of an aqueous sample is critical in a number of industries such as pharmaceuticals, biomedical, water supply, and other manufacturing fields. Measurement of pH may allow for proper treatment of water or ensuring proper water quality for sensitive purposes, and allows for identifying the overall quality of the water. One method to measure pH in an aqueous sample includes the use of electrodes which require constant maintenance and calibration of the pH measurement system.

### BRIEF SUMMARY

In summary a method is provided for manufacturing a boron-doped diamond (BDD) pH electrode having a pH sensitive carbon region including sp2 oxidized carbon structures and quinone or quinone-like groups, comprising the steps described at claim 1. The dependent claims outline advantageous ways of carrying out the method.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention will be pointed out in the appended claims.

### BRIEF DESCRIPTION OF THE SEVERAL VIEWS OF THE DRAWINGS

FIG. 1 illustrates a schematic diagram of pulse overlap in an example embodiment.
FIG. 2 A - C illustrates a schematic diagram of a pattern in an example embodiment.
FIG. 3 A - C illustrates a schematic diagram of another pattern in an example embodiment.
FIG. 4 illustrates an example of computer circuitry.

### DETAILED DESCRIPTION

The measurement of the pH of water or other aqueous solutions or samples is common and allows for determination of the quality or other characteristics of the aqueous solution. Conventional pH electrodes for measurement of pH may be constructed using fragile, thin glass. This glass breaks easily leading to higher replacement and maintenance costs. The possible breakage of glass pH electrodes may also limit their use in food and beverage applications. Conventional pH electrodes also may have "alkali errors." These errors arise from interfering ions such as sodium and potassium affecting the pH response at high pH values. A commercial need exists for a robust pH measurement electrode that requires less maintenance while maintaining measurement of pH in sample containing heavy metals or a low conductivity sample.

Another method of measuring pH of an aqueous sample uses a laser machined boron-doped diamond (BDD) material to create a sensor capable of measuring pH. Laser machining of the BDD creates pH sensitive quinone-like structures on the BDD material, due to the introduction of sp2 carbon. These laser machined areas or pits may be in an array pattern. In other words, the laser machined areas may be a series of spots across a surface. The laser machining of these multiple spots may be challenging. For example, an array of laser machines pits across a surface requires using the laser repeatedly across the surface. Repeating machining of a BDD surface with a laser may create heat. The generation of heat may lead to further creation of sp2 carbon, and subsequent quinone-like structures capable of sensing pH. Repeated laser machining also requires laser pulses which may be time consuming in the manufacturing process. A proper control of a laser machining may maintain a proper aspect ratio of features to prevent fouling or blocking of an electrode surface.

A BDD electrode gives greater accuracy, especially improved accuracy in samples with low conductivity or low buffer capacity. BDD electrodes may measure pH in all pH ranges including an environmentally relevant range of pH 4 to 11. A better control over quinone-like structure generation is required as the quinone-like structures may impact accuracy in low conductive and low buffer capacity solutions. Heating from a laser machining process may lead to greater quinone-like structures. What is needed is a method to control production of quinone-like structures and/or reduce heating during a laser machining. Additionally, the method may reduce manufacturing time.

Accordingly, method as described herein is used to make an electrode. The electrode is a BDD electrode. The electrode is sensitive to pH. The pH sensitive region is laser machined. The laser machining creates a carbon region. The carbon region is a sp2 carbon region. The carbon region has oxidized carbon structures and or quinone-like groups. In an embodiment, the method determines a pulse overlap modification. A pulse overlap modification refers to the manner in which laser pulses overlap or do not overlap. The pulse overlap modification may be 2-dimensional, 1-dimensional, and/or 0-dimensional, as described below. In an embodiment, the method selects a pattern for machining a carbon region. For example, the pattern may be an array, parallel line, lines, concentric circle, or the like. Other patterns are described and disclosed. A laser machined electrode determines a pH by identifying an electrical potential of an aqueous sample.

The electrode has a carbon region and a pH sensitive carbon region. For example, the primary electrode is a BDD electrode with a plurality of sp2 carbon regions. The primary electrode may be a working electrode. The pH sensitive carbon region is laser machined. The carbon region comprises sp2 carbon materials that can include diamond-like materials doped with elements like boron (BDD). The pH sensitive carbon region is an sp2 carbon region that is included on a boron doped diamond-based pH electrode. Being included means that the sp2 carbon region is introduced into, integrated into, contained within, laser micro machined into, or otherwise integrated into the boron doped diamond electrode. In other words, while the sp2 carbon region and the boron doped diamond are integrated into the same electrode, they are chemically different regions of the electrode. The carbon region has oxidized carbon structures. The oxidized carbon structure has quinone or quinone-like groups.

The electrode produced by a described method may also be used with at least one reference electrode and at least one auxiliary electrode. In an embodiment, an applied potential protocol is applied to a first measurement electrode. An electrical potential between the measurement electrode and a reference electrode is measured. In an embodiment, the electrical potential corresponds to a pH measurement of an aqueous sample. The measurement electrode, at least one reference electrode, and at least one auxiliary electrode are operatively coupled to circuitry to measure, analyze, and store pH measurements of a sample.

The use of BDD serves as a better electrode material than other carbon-based or metallic materials (e.g., silver, gold, mercury, nickel, etc.) because these materials may be more electrocatalytically active, and may generate interfering signals contributing to the errors in the measurement of pH.

The illustrated example embodiments will be best understood by reference to the figures. The following description is intended only by way of example, and simply illustrates certain example embodiments.

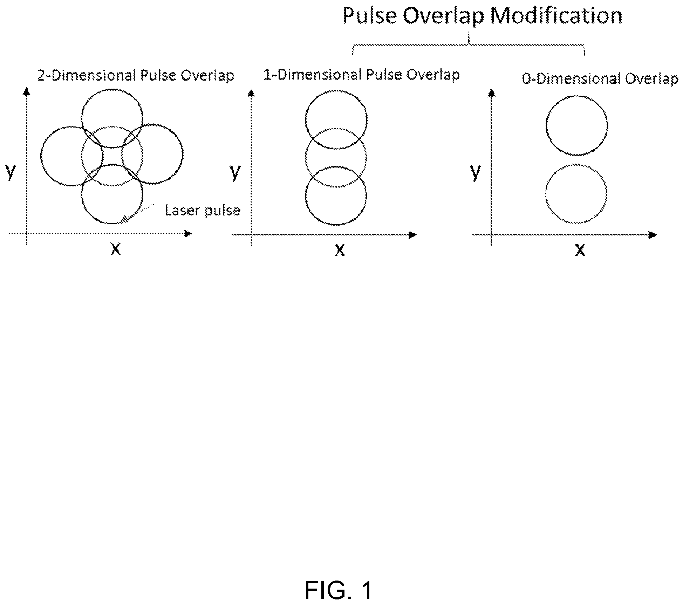

Referring now to FIG. 1, an embodiment determines a pulse overlap modification for an electrode. The pulse overlap modification refers to the method in which an electrode is laser machined or laser pulsed to produce a functional area upon the electrode face. The electrode is a BDD electrode. BDD electrode reduces fragility, alkali errors, wet storage requirements, or the like as compared to glass electrode.

The electrode measures pH of a sample. The electrode is laser micromachined or machined to introduce an array of pits into the electrode surface or face. A combination of pits creates a pattern upon the electrode. The laser machining introduces sp2 carbon upon the electrode. The sp2 carbon includes quinone or quinone-like groups which undergo proton-coupled electron transfer. The laser machined electrodes are used to perform electrochemical measurements to measure a pH response on the electrode in which the observed peak may be indicative of a pH of a sample.

A BDD pH electrode operates in a Nernstian manner across a pH range in a buffered solution. In an unbuffered solution, a BDD pH electrode deviates from an expected theoretical response. A deviation is linked to an amount of sp2 carbon present and/or the amount of quinone-like groups on an electrode surface. Thus, control of carbon region machining is critical to the efficient manufacture of an accurate electrode. The method disclosed provides better manufacturing of a suitable BDD pH electrode with sp2 regions.

A pulse overlap modification is determined to laser machine a BDD pH electrode. A 2-dimensional machining process is used to manufacture a BDD pH electrode. A 2-dimensional machining is characterized when three or more laser pulses overlap. A 2-dimensional machining may result in a larger area of graphitization. A 2-dimensional machining results in a creation of sp2 carbon within a BDD surface. A 2-dimensional machining produces sites upon the BDD that are machined more than once (re-machining). Re-machining leads to an increase of sp2 carbon, and creates a different distribution of quinone as compared to a single pulse or single laser machining. Re-machining before cooling down of the electrode surface leads to the creation of more sp2 carbon. Re-machining is inefficient and leads to longer manufacturing times.

In an embodiment, a different pulse overlap modification is determined. In other words, laser machining parameters are modified such that pulse overlap, which may be referred to as pitch, is adjusted. The pulse overlap is adjusted such that a single spot on the surface is machined no more than twice. For example, an area may be laser machined with a 1-dimensional pulse overlap modification. A 1-dimensional pulse overlap modification may be used to laser machine a line, hash, circle, linear shapes that may be rastered, or the like.

In an embodiment, a 0-dimensional pulse overlap is used to manufacture an electrode. In other words, a 0-dimensional pulse overlap have no overlap of laser pulses. A 0-dimensional overlap allows a laser machining method to machine a surface only once. In other words, the laser machining of an sp2 carbon region into the BDD surface may be controlled.

Laser machining heats the electrode surface. A pulse overlap is selected to minimize or mitigate the heating of an electrode surface. A determination of a pulse overlap also facilitates a faster and/or more controlled laser machining process. For example, less pulse overlap reduces multiple laser pulses over one area of an electrode and allows laser machining in a more efficient manner.

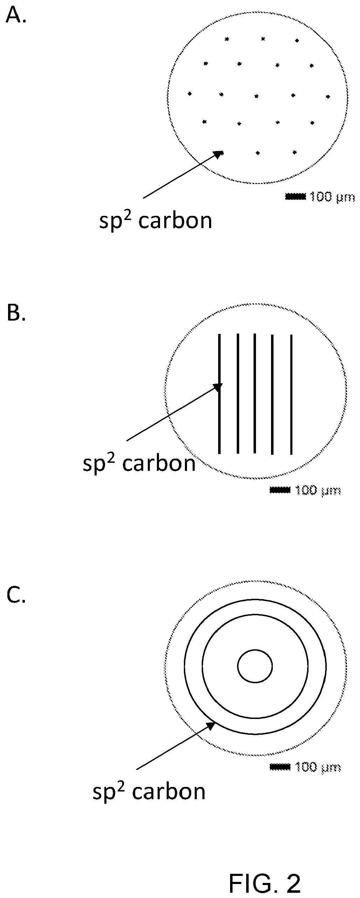

Referring to FIG. 2, example embodiments of a pattern may be selected. A pattern refers to the shape or pattern of laser machined areas upon the electrode surface. A pattern may refer to an array, one or more lines, circles, or the like. In other words, a BDD electrode is laser machined in one or more areas to produce a sp2 carbon area. The sp2 carbon area may be functionalized to be pH sensitive, as an example. In an embodiment, an array is laser machined (FIG. 2A). An array may be a plurality of laser machined spots, areas, pits, or the like across an electrode surface. The plurality of laser machined areas may be of a uniform diameter, varying diameter, evenly spaced, spaced, or the like based upon an electrode application or use.

In an embodiment, at least one line is laser machined (FIG. 2B). There may be one line or two or more lines laser machined. The lines may be parallel or non-parallel. The lines may be a uniform or different length and/or width. The laser machining may not be a straight line. For example, the laser machining may be a curved section, S-shaped, geometric shapes, or the like.

In an embodiment, a circle or oval like shape may be laser machined (FIG. 2C). The circles or oval may be concentric or non-concentric. The spacing and placement of the circles or ovals may be uniform or different. Any combination of laser machining upon an electrode may be performed.

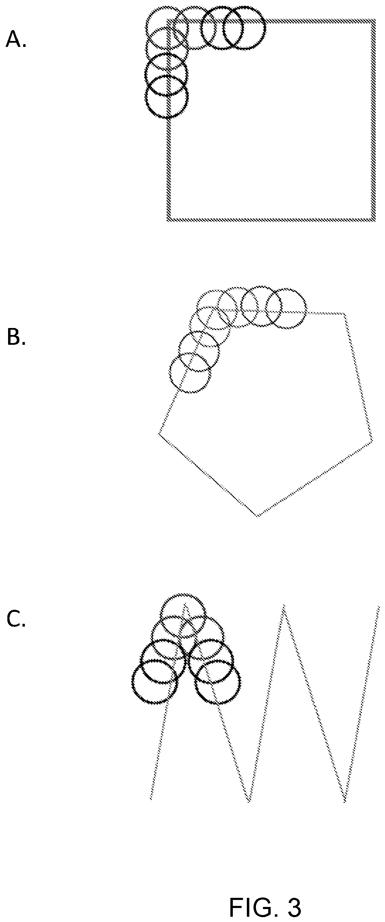

Referring to FIG. 3, example embodiments of a pulse overlap and a pattern are illustrated. For example, a laser machining of a square (FIG. 3A), a pentagon (FIG. 3B), and a zig-zag (FIG. 3C) pattern are illustrated. In an embodiment, pulse overlap modification is used to reduce the re-machining using laser pulses upon the electrode. Despite a reduction of pulse overlap, there may still be pulse overlap. Pulse overlap may occur in a location such as a corner of a geometric shape. For example, if a system determines a 1-dimensional pulse overlap, a 2-dimensional pulse overlap may occur as the machining process changes direction such as turning a corner. As another example, a more acute angle on a shape may create a greater pulse overlap as compared to an obtuse angle of a shape. Thus, while an embodiment may prefer a 1-dimensional or 0-dimensional pulse overlap, a greater pulse overlap may occur at some areas. This greater pulse overlap may be a very small amount of area as compared to the entire laser machined surface area upon the electrode. The system reduces laser pulses in a corner, bend, turn, or the like to reduce pulse overlap.

The laser machining may be tailored for a specific application. For example, pulse overlap may be reduced to reduce a heating of the electrode material. Heat may cause damage of an electrode, reduction of tolerances, reduction of accuracy, or the like. The laser machining may be adjusted. Parameters that may be adjusted include, but are not limited to: size, diameter, number, size, shape, length of time of machining, speed, depth, or the like of a laser pulse. A laser machining may employ different parameters on the same or different electrodes.

The method and system may control the laser machining process. For example, the system may control the x - y coordinates of the laser pulse upon an electrode, the length of time of a pulse, speed of movement of the laser, latency between pulses, pulse overlap, pattern type, diameter, power, depth, or the like. The system may laser machine one or more electrodes either sequentially or in parallel. The system may be preprogrammed either from an input of instruction by a user or from a stored database upon the device, cloud, or the like. The system may adjust parameters to fit the needs of a user, application, quality control protocols, or the like.

The laser machined electrode may be used for a pH measurement, interferent measurement, electrochemical measurement, or the like. The system may have at least one reference electrode. The system may have at least one auxiliary electrode. The system may comprise an electrode that is a BDD electrode with at least one sp2 carbon region laser machined upon a face of the electrode. The method and system may comprise an additional electrode. The additional electrode may be a BDD electrode with no sp2 region or an sp2 region with a lesser area than the sp2 area of a primary electrode. The laser machined electrode may be a part of a 3-electrode system. For example, the system may measure using an auxiliary, a reference, and either a primary or secondary electrode.

The laser machined electrode may also be used with at least one reference electrode and one auxiliary electrode. An applied potential protocol may be utilized and a resultant electrical potential may be measured between the at least one measurement electrode and at least one reference electrode. The systems and methods as described herein provide a technique for accurate measurement of pH in a range of sample types such as samples with low buffer capacity or samples with low conductivity.

The carbon region may be of a BDD material, BDD sp2 material, quinone structures, quinone-like structures, oxidized carbon structures, or the like. The carbon material is laser machined upon the measurement electrode. The measurement electrode may be a BDD electrode. The laser machining may be a plurality of laser machined areas or a continuous region. The laser machined region may be circular or circular-like in shape such as a circle, oval, or the like. In other words, other shapes of carbon regions are disclosed and may be laser machined to a particular use or configuration of a measurement electrode.

The size, depth, or surface area of the laser machined sp2 region may be constructed for a particular use. In other words, the sp2 region may be machined in accordance with actual or expected pH ranges, conductivity range, buffer capacity, heavy metal type, heavy metal concentration, size of particles or components, or the like within a sample to be measured. The sp2 carbon regions may be of a different number and/or diameter. For example, the diameter of a single sp2 laser machined area may be larger or smaller. As another example, the number of sp2 regions may be increased or decreased on a given electrode. The diameter and number of sp2 regions may be increased or decreased upon an electrode.

The laser machined electrodes may be fully or at least partially disposed in the volume of aqueous solution. For example, if the aqueous solution is introduced into a chamber having one or more electrodes, the aqueous solution may at least partially cover the one or more electrodes. As another example, the one or more electrodes may be partially disposed within the chamber with the other portion of the electrode outside the chamber. Thus, when the aqueous solution is introduced into the chamber it only covers the portion of the electrodes that are within the chamber.

The laser machined electrode may measure an electrical potential of the volume of aqueous sample across at least one measurement electrode and at least one reference electrode. A measurement system may have either, one measurement electrode and one reference electrode, or have a plurality of measurement electrodes and a plurality of reference electrodes.

The electrical potential may be measured across one or more electrodes, for example, a series of electrodes. The primary measurement electrode may be used to measure the electrical potential attributable to a pH of an aqueous sample. The measurement electrode may contain a carbon region. The carbon region is described above. The carbon region is laser machined The carbon region may be a BDD, BDD sp2, oxidized carbon structure, quinone, quinone-like structure or the like. The carbon region may be a continuous region in a circular-like shape, a line, a geometric shape, or the like.

The various embodiments described herein thus represent a technical improvement to conventional methods and instrument for pH measurement. Using the techniques as described herein, an embodiment may use a method and device for an instrument for pH measurement. This is in contrast to conventional methods with limitations mentioned above. Such techniques provide a better method to construct an instrument for pH measurement.

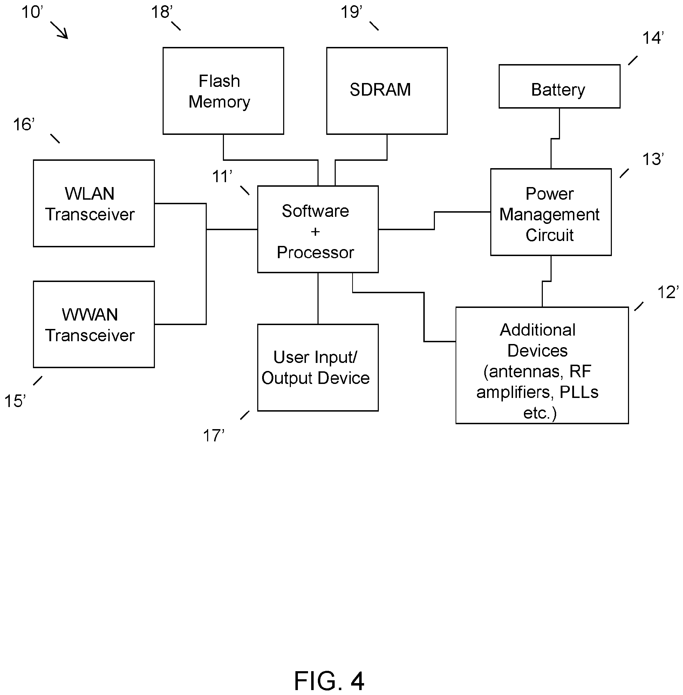

While various other circuits, circuitry or components may be utilized in information handling devices, with regard to an instrument for pH measurement according to any one of the various embodiments described herein, an example is illustrated in FIG. 4. Device circuitry 10' may include a measurement system on a chip design found, for example, a particular computing platform (e.g., mobile computing, desktop computing, etc.) Software and processor(s) are combined in a single chip 11'. Processors comprise internal arithmetic units, registers, cache memory, busses, I/O ports, etc., as is well known in the art. Internal busses and the like depend on different vendors, but essentially all the peripheral devices (12') may attach to a single chip 11'. The circuitry 10' combines the processor, memory control, and I/O controller hub all into a single chip 11'. Also, systems 10' of this type do not typically use SATA or PCI or LPC. Common interfaces, for example, include SDIO and I2C.

There are power management chip(s) 13', e.g., a battery management unit, BMU, which manage power as supplied, for example, via a rechargeable battery 14', which may be recharged by a connection to a power source (not shown). In at least one design, a single chip, such as 11', is used to supply BIOS like functionality and DRAM memory.

System 10' typically includes one or more of a WWAN transceiver 15' and a WLAN transceiver 16' for connecting to various networks, such as telecommunications networks and wireless Internet devices, e.g., access points. Additionally, devices 12' are commonly included, e.g., a transmit and receive antenna, oscillators, PLLs, etc. System 10' includes input/output devices 17' for data input and display/rendering (e.g., a computing location located away from the single beam system that is easily accessible by a user). System 10' also typically includes various memory devices, for example flash memory 18' and SDRAM 19'.

It can be appreciated from the foregoing that electronic components of one or more systems or devices may include, but are not limited to, at least one processing unit, a memory, and a communication bus or communication means that couples various components including the memory to the processing unit(s). A system or device may include or have access to a variety of device readable media. System memory may include device readable storage media in the form of volatile and/or nonvolatile memory such as read only memory (ROM) and/or random access memory (RAM). By way of example, and not limitation, system memory may also include an operating system, application programs, other program modules, and program data. The disclosed system may be used in an embodiment of an instrument for pH measurement.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions stored on a device readable storage medium such as a non-signal storage device, where the instructions are executed by a processor. In the context of this document, a storage device is not a signal and "non-transitory" includes all media except signal media.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., near-field communication, or through a hard wire connection, such as over a USB connection.

Example embodiments are described herein with reference to the figures, which illustrate example methods, devices and products according to various example embodiments. It will be understood that the actions and functionality may be implemented at least in part by program instructions. These program instructions may be provided to a processor of a device, e.g., a measurement device such as illustrated in FIG. 1, or other programmable data processing device to produce a machine, such that the instructions, which execute via a processor of the device, implement the functions/acts specified.

It is noted that the values provided herein are to be construed to include equivalent values as indicated by use of the term "about." The equivalent values will be evident to those having ordinary skill in the art, but at the least include values obtained by ordinary rounding of the last significant digit.

## Claims

1. A method for manufacturing a boron-doped diamond (BDD) pH electrode with having a pH sensitive carbon region including sp2 oxidized carbon structures and quinone or quinone-like groups, comprising:
selecting a pattern for machining with laser pulses the carbon region with an array of laser machined spots across an electrode surface;
modifying laser machining parameters adjusting a pulse overlap such that a single spot on the surface is machined no more than twice;
machining the sp2 carbon region with the modified laser machining parameters in the selected pattern by pulsing a laser onto a the BDD electrode surface in the selected pattern, thereby reducing heating of the electrode material in the pulse overlap area.

2. The method of claim 1, wherein the parameters being adjusted include size, and/or number, and/or shape, and/or length of machining, and/or speed, and/or depth of a laser pulse.

3. The method of claim 1, wherein the pulse laser machining process is configured to control the X-Y coordinates of a laser pulse upon the electrode, and/or a length time of a pulse, and/or a speed movement of the laser, and/or a latency between pulses, and/or a pulse overlap, and/or a pattern type.

4. The method of claim 1, wherein the pattern is a plurality of spots upon the BDD electrode surface.

5. The method of claim 1, wherein the pattern is a plurality of parallel lines upon the BDD electrode surface.

6. The method of claim 1, wherein the pattern is a plurality of concentric circles.

7. The method of claim 1, wherein the modifying laser machining parameters adjusting a pulse overlap is based upon the pattern selected such that a single spot on the surface is machined no more than twice.

8. The method of claim 1, wherein the laser machining of the BDD electrode surface is creates pH sensitive quinone-like structures on the boron-doped diamond (BDD) material, due to the introduction of sp2 carbon.

9. The method of claim 1, wherein modifying laser machining parameters adjusting a pulse overlap such that a single spot on the surface is machined no more than twice is used to reduce re-machining using laser pulses upon the electrode.

10. The method of claim 1, wherein modifying laser machining parameters adjusting a pulse overlap such that a single spot on the surface is machined no more than twice is used to reduce laser pulses in a corner, bend, turn or the like of the selected pattern, thereby reducing a heating of the electrode material.

## Patentansprüche

1. Verfahren zur Herstellung einer Bor-dotierten Diamant (BDD)-pH-Elektrode mit einem pH-empfindlichen Kohlenstoffbereich, der oxidierte sp2-Kohlenstoffstrukturen und Chinon- oder chinonartige Gruppen enthält, umfassend:
Auswählen eines Musters zur Bearbeitung des Kohlenstoffbereichs mit Laserpulsen mit einer Anordnung von laserbearbeiteten Punkten über eine Elektrodenoberfläche;
Modifizieren der Laserbearbeitungsparameter, Anpassen einer Impulsüberlappung, sodass ein einzelner Punkt auf der Oberfläche nicht mehr als zweimal bearbeitet wird;
Bearbeiten des sp2-Kohlenstoffbereichs mit den modifizierten Laserbearbeitungsparametern im ausgewählten Muster durch Pulsieren eines Lasers auf die Oberfläche der BDD-Elektrode im ausgewählten Muster, wodurch die Erwärmung des Elektrodenmaterials im Impulsüberlappungsbereich reduziert wird.

2. Verfahren nach Anspruch 1, wobei die angepassten Parameter die Größe und/oder Anzahl und/oder Form und/oder Länge der Bearbeitung und/oder Geschwindigkeit und/oder Tiefe eines Laserpulses umfassen.

3. Verfahren nach Anspruch 1, wobei das Impulslaser-Bearbeitungsverfahren so konfiguriert ist, dass es die X-Y-Koordinaten eines Laserimpulses auf der Elektrode und/oder eine Impulsdauer und/oder eine Geschwindigkeitsbewegung des Lasers und/oder eine Latenz zwischen Impulsen und/oder eine Impulsüberlappung und/oder einen Mustertyp steuert.

4. Verfahren nach Anspruch 1, wobei das Muster aus einer Vielzahl von Punkten auf der BDD-Elektrodenoberfläche besteht.

5. Verfahren nach Anspruch 1, wobei das Muster aus einer Vielzahl paralleler Linien auf der BDD-Elektrodenoberfläche besteht.

6. Verfahren nach Anspruch 1, wobei das Muster aus mehreren konzentrischen Kreisen besteht.

7. Verfahren nach Anspruch 1, wobei die modifizierenden Laserbearbeitungsparameter, die eine Impulsüberlappung einstellen, auf dem ausgewählten Muster basieren, so dass ein einzelner Punkt auf der Oberfläche nicht mehr als zweimal bearbeitet wird.

8. Verfahren nach Anspruch 1, wobei die Laserbearbeitung der BDD-Elektrodenoberfläche pH-empfindliche, Chinon-ähnliche Strukturen auf dem bordotierten Diamantmaterial (BDD) erzeugt, und zwar aufgrund der Einführung von sp2-Kohlenstoff.

9. Verfahren nach Anspruch 1, wobei modifizierte Laserbearbeitungsparameter, die Impulsüberlappung so einstellen, dass ein einzelner Punkt auf der Oberfläche nicht mehr als zweimal verwendet wird, um die Nachbearbeitung unter Verwendung von Laserimpulsen auf der Elektrode zu reduzieren.

10. Verfahren nach Anspruch 1, wobei modifizierte Laserbearbeitungsparameter, die eine Impulsüberlappung so einstellen, dass ein einzelner Punkt auf der Oberfläche nicht mehr als zweimal bearbeitet wird, verwendet werden, um Laserimpulse in einer Ecke, Biegung, Drehung oder dergleichen des ausgewählten Musters zu reduzieren, wodurch eine Erwärmung des Elektrodenmaterials reduziert wird.

## Revendications

1. Procédé de fabrication d'une électrode de pH en diamant dopé au bore (BDD) ayant une région de carbone sensible au pH comprenant des structures de carbone oxydées sp2 et des groupes de quinone ou de type quinone, comprenant :
sélectionner un modèle pour l'usinage avec des impulsions laser de la région de carbone avec un réseau de points usinés au laser sur une surface de l'électrode ;
modifier les paramètres d'usinage au laser en ajustant un chevauchement des impulsions de manière à ce qu'un seul point de la surface ne soit pas usiné plus de deux fois ;
usiner la région de carbone sp2 avec les paramètres modifiés d'usinage au laser dans le motif sélectionné, en envoyant des impulsions laser sur la surface de l'électrode BDD dans le motif sélectionné, réduisant ainsi le chauffage du matériau de l'électrode dans la zone de chevauchement des impulsions.

2. Procédé selon la revendication 1, dans lequel les paramètres étant ajustés comprennent la taille, et/ou le nombre, et/ou la forme, et/ou la longueur de l'usinage, et/ou la vitesse, et/ou la profondeur d'une impulsion laser.

3. Procédé selon la revendication 1, dans lequel le processus d'usinage par laser à impulsions est configuré pour contrôler les coordonnées X-Y d'une impulsion laser sur l'électrode, et/ou une durée d'une impulsion, et/ou une vitesse de déplacement du laser, et/ou une latence entre les impulsions, et/ou un chevauchement des impulsions, et/ou un type de motif.

4. Procédé selon la revendication 1, dans lequel le motif est une pluralité de points sur la surface de l'électrode BDD.

5. Procédé selon la revendication 1, dans lequel le motif est une pluralité de lignes parallèles sur la surface de l'électrode BDD.

6. Procédé selon la revendication 1, dans lequel le motif est une pluralité de cercles concentriques.

7. Procédé selon la revendication 1, dans lequel la modification des paramètres d'usinage laser ajustant un chevauchement d'impulsions est basée sur le motif sélectionné de manière à ce qu'un seul point de la surface ne soit pas usiné plus de deux fois.

8. Procédé selon la revendication 1, dans lequel l'usinage laser de la surface de l'électrode BDD crée des structures de type quinone sensibles au pH sur le matériau de diamant dopé au bore (BDD), en raison de l'introduction de carbone sp2.

9. Procédé selon la revendication 1, dans lequel la modification des paramètres d'usinage laser ajustant un chevauchement d'impulsions de telle sorte qu'un seul point de la surface ne soit pas usiné plus de deux fois est utilisée pour réduire le ré-usinage à l'aide d'impulsions laser sur l'électrode.

10. Procédé selon la revendication 1, dans lequel la modification des paramètres d'usinage au laser ajustant le chevauchement des impulsions de manière à ce qu'un seul point de la surface ne soit pas usiné plus de deux fois est utilisée pour réduire les impulsions laser dans un coin, un coude, un virage ou autre du motif sélectionné, réduisant ainsi l'échauffement du matériau de l'électrode.
